(19) Europäisches Patentamt
European Patent Office
Office européen des brevets

(11) **EP 3 083 983 B1**

(12) **EUROPEAN PATENT SPECIFICATION**

(45) Date of publication and mention
of the grant of the patent:
**23.10.2019 Bulletin 2019/43**

(21) Application number: **14871421.5**

(22) Date of filing: **15.12.2014**

(51) Int Cl.:
*C12Q 1/22* (2006.01)     *B63B 43/06* (2006.01)
*B63B 57/00* (2006.01)    *B63J 4/00* (2006.01)
*C02F 1/32* (2006.01)     *C12Q 1/06* (2006.01)
*C12Q 1/18* (2006.01)     *G01N 33/52* (2006.01)
*C12Q 1/02* (2006.01)

(86) International application number:
**PCT/CA2014/000890**

(87) International publication number:
**WO 2015/089631 (25.06.2015 Gazette 2015/25)**

(54) **METHOD FOR ASSAYING FOR LOSS OF AN ORGANISM IN AN AQUEOUS LIQUID**

VERFAHREN ZUR UNTERSUCHUNG AUF VERLUST EINES ORGANISMUS IN EINER WÄSSRIGEN FLÜSSIGKEIT

PROCÉDÉ POUR DOSER LA PERTE D'UN ORGANISME DANS UN LIQUIDE AQUEUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2013 US 201361963982 P**

(43) Date of publication of application:
**26.10.2016 Bulletin 2016/43**

(73) Proprietor: **Trojan Technologies**
London, ON N5V 4T7 (CA)

(72) Inventors:
• **MACINTYRE, Hugh Logan**
Dartmouth, Nova Scotia B3A 3R2 (CA)
• **CULLEN, John Joseph**
Barss Corner, Nova Scotia B0R 1A0 (CA)

(74) Representative: **Sandri, Sandro**
**Bugnion S.P.A.**
**Via Pancaldo 68**
**37138 Verona (IT)**

(56) References cited:
• P M EKER A: "Photorepair Processes", MOLECULAR MODELS OF PHOTORESPONSIVENESS PART OF THE NATO ADVANCED SCIENCE INSTITUTES SERIES BOOK SERIES (NSSA, VOLUME 68), G. MONTAGNOLI, B.F. ERLANGER, vol. 68, 1 January 1983 (1983-01-01), pages 109-132, XP008185068, DOI: 10.1007/978-1-4757-0896-7_8 ISBN: 978-0-306-41472-5
• D.E. PEGG: "Viability assays for preserved cells, tissues, and organs", CRYOBIOLOGY., vol. 26, no. 3, 1 June 1989 (1989-06-01), pages 212-231, XP055388982, US ISSN: 0011-2240, DOI: 10.1016/0011-2240(89)90016-3
• F. BOSSHARD ET AL: "Solar disinfection (SODIS) and subsequent dark storage of Salmonella typhimurium and Shigella flexneri monitored by flow cytometry", MICROBIOLOGY, vol. 155, no. 4, 1 April 2009 (2009-04-01) , pages 1310-1317, XP055388837, GB ISSN: 1350-0872, DOI: 10.1099/mic.0.024794-0
• DRING ET AL.: 'Sensitivity of intertidal and subtidal red algae to UVA and UVB radiation , as monitored by Chlorophyll fluorescence measurements: Influence of collection depth and season, and length of irradiation' EUROPEAN JOURNAL OF PHYCOLOGY vol. 31, no. 4, 1996, pages 293 - 302, XP055310230

- SATO M. ET AL.: 'A Simple and Rapid Dual-fluorescence Viability Assay for Microalgae' MICROBIOLOGY AND CULTURE COLLECTIONS vol. 20, no. 2, December 2004, pages 53 - 59, XP055310232
- DORSEY J. ET AL.: 'Rapid Analytical Technique for the Assessment of Cell Metabolic Activity in Marine Microalgae' CYTOMETRY vol. 10, no. 5, 1989, pages 622 - 628, XP055310235
- CARNEY, K. J.: 'Marine bioinvasion prevention: understanding ballast water transportation conditions and the development of effective treatment systems' A THESIS SUBMITTED FOR THE AWARD OF DOCTE OF PHILOSOPHY (PHD) AT NEWCASTLE UNIVERSITY July 2011, SCHOOL OF MARINE SCIENCE AND TECHNOLOGY NEWCASTLE UPON TYNE, XP055310240 Retrieved from the Internet: <URL:https://theses.ncl.ac.uk/dspace/handle /1 0443/1246> [retrieved on 2015-03-20]

**Description**

CROSS-REFERENCE TO RELATED APPLICATION

The present application claims the benefit under 35 U.S.C. § 119(e) of provisional patent application S.N. 61/963,982, filed December 20, 2013.

BACKGROUND OF THE INVENTION

FIELD OF THE INVENTION

In a general aspect, the present invention relates to a method for assaying for loss of a target microorganism comprised in water as defined in claim 1.

DESCRIPTION OF THE PRIOR ART

It is known in the art that aqueous liquids (e.g., municipal wastewater, municipal drinking water, industrial effluents, ballast water on shipping vessels, etc.) can be disinfected of microorganisms using a variety of treatments that lead to immediate or delayed mortality. Treatment with appropriate dosages of ultraviolet radiation (UVR), such as ultraviolet-C (UV-C) radiation, leads to immediate sub-lethal effects resulting in delayed mortality and reproductive impairment. Generally, these effects can only be assessed directly using time-consuming culture-based growth experiments that may take days to months to complete.

Treatment of ballast water on shipping vessels is regulated by the United Nations International Marine Organization (IMO) and the United States Coast Guard (USCG). The USCG has recommended (ETV 2010) that the effectiveness of treatment be assessed using the vital stains fluorescein diacetate (FDA) and 5-chloromethylfluorescein diacetate (CMFDA). Vital stains, therefore, are believed to represent the conventional approach for rapid assessment of ballast water treatment effectiveness.

Fluorescein-based vital stains (e.g., FDA and CMFDA) assay the integrity of the cell membrane and the functionality of esterases in the cells being tested. They have many deficiencies when used to assay viability in phytoplankton. For example, the staining

- is time-dependent (Dorsey et al. 1989);
- is highly variable between species (Selvin et al. 1988; Murphy and Cowles 1997; Onji et al. 2000; Agustí and Sanchez 2002; Garvey et al. 2007; Peperzak and Brussaard 2011);
- varies with growth phase within a species (Gilbert et al. 1992; Garvey et al. 2007); and
- can be masked and confounded by green autofluorescence (Tang and Dobbs, 2007; Steinberg et al. 2011) and, in some cases, red chlorophyll autofluorescence (Agustí and Sanchez 2002; Garvey et al. 2007).

Neither cell membranes nor esterases are the primary targets of UVR damage. The primary cause of mortality from UV-C treatment is believed to be through damage to nucleotides (Gieskes and Buma 1997; Sinha and Hader 2002). The damage (e.g., dimerization of the nucleotides in DNA and RNA) interferes with nucleotide replication and transcription for the synthesis of proteins. This damage is not detected by FDA/CMFDA staining.

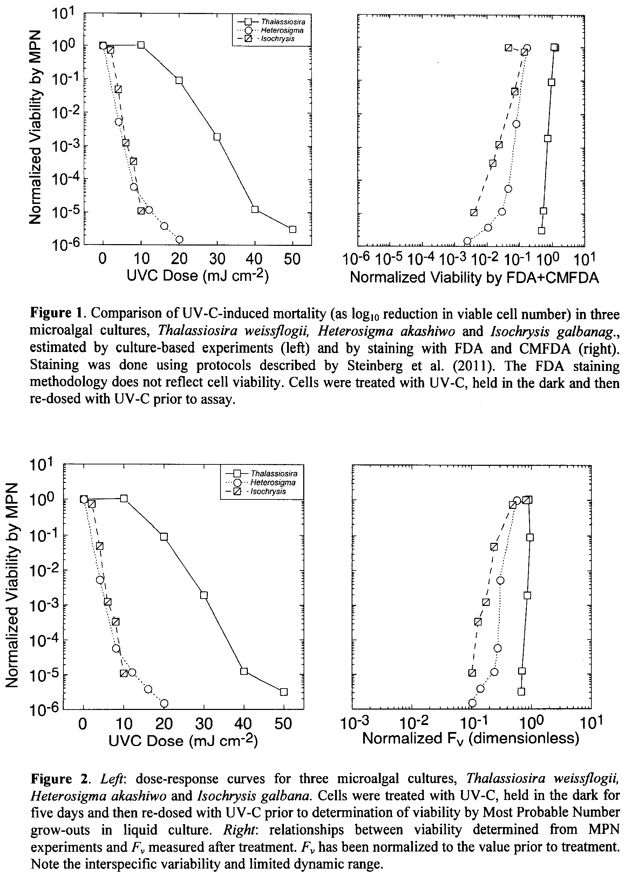

Consequently, an assay for UV-C-based mortality based on FDA is inaccurate because of a high rate of false positive results: cells that are successfully treated and incapable of reproduction can retain intact membranes and functional esterases and thus stain heavily with FDA. Although incapable of growth and reproduction (functionally non-viable in the natural environment), they are assessed as being healthy - see Figure 1.

The purpose of ballast water treatment is to prevent the introduction of potentially invasive microorganisms; this can be accomplished by killing them or making them non-viable, i.e., incapable of reproduction and thus unable to colonize receiving waters.

Treatment with UVR, a proven technology for municipal drinking water and municipal wastewater disinfection has been described for ballast water applications - see, for example, International Patent Publication Number WO 2010/130031 [Fraser] and International Patent Publication Number WO 2012/061924 [DaCosta et al.]. However, since UVR acts primarily through reproductive impairment and delayed mortality rather than through immediate killing, assessment of the technology for microorganisms is difficult. Broadly, this is for two reasons:

- *Techniques for measuring reproduction directly are time-consuming and subject to uncertainty when applied to mixed assemblages in natural samples* - The direct measure of a microorganism's viability is the ability to reproduce, i.e., grow in culture (as determined with so-called grow-out or regrowth methods, e.g., Liebich et al. 2012). In principle, culture-based methods such as the Most Probable Numbers (MPN) technique provide the gold-standard assessment of viability (cf. Throndsen 1978). They require days to months to perform, though, and are thereby unsuitable for routine verification of ballast water treatment compliance of a given shipping vessel. Further, when applied on naturally-occurring plankton assemblages, uncertainties are introduced because some aquatic microorganisms (including heterotrophs for which culture conditions are not designed) cannot be cultured reliably. Therefore, the effects of UVR on their viability cannot be reliably measured directly using MPN.

- *The mode of action of UVR differs from those of other disinfection technologies, so commonly used "live* vs. *dead" assays greatly underestimate the effectiveness of UVR in preventing the introduction of invasive microorganisms -* Damage to DNA (e.g., formation of pyrimidine dimers) is the principal reason why UVR treatment inactivates microbes (Gieskes and Buma 1997; Sinha and Hader 2002). Cells that have been rendered nonviable by UVR treatment can retain some metabolic function and thereby appear as living when assessed with vital stains that are currently used to measure the effectiveness of ballast water treatment (ETV 2010) - see Figure 1. Consequently, ballast water treatment guidelines that are based on validation with vital stains - i.e., "living" as determined with vital stains, rather than "viable", defined as the ability to reproduce - can impose inappropriately high design doses that would result in the need for larger treatment systems with consequential greater energy demand.

In light of the above, reliable and rapid alternatives to culture-based assays are needed, but existing approaches based on vital stains do not reliably detect delayed mortality and reproductive impairment from UVR treatment. Assessments of damage to photosynthetic systems, based on measurements of chlorophyll fluorescence, can detect damage due to UVR, but measures of photodamage alone do not reliably indicate mortality or reproductive impairment, in part because the molecular targets associated with damage to photosystems are not the same as for DNA replication and protein synthesis.

Therefore, there is a pressing need for a rapid assay of more general metabolic damage. Preferably, such an assay would correlate with reproductive impairment as determined through culture-based assays of viability.

SUMMARY OF THE INVENTION

The present invention provides a method for assaying for loss of viability of a microorganism as defined in claim 1.

Preferred embodiments are defined in dependent claims 2-9.

The present inventors have discovered that measurements of damage to the photosynthesis repair process serve as good proxies for generalized metabolic impairment and the loss of viability of an organism, preferably a microorganism. Thus, the present inventors have developed a rapid assay of damage to photosynthetic systems, and repair of that damage, preferably based on measurements of fluorescence, preferably variable fluorescence, most preferably variable chlorophyll fluorescence. The present inventors have established that an index based on such measurements can be used to reliably predicted survivorship in photosynthetic microorganisms treated with UV-C. By extending fluorescence-based assays of photodamage to quantify both damage to photosystems and its repair (which is dependent on protein synthesis), a rapid and sensitive assessment of general metabolic impairment and loss of viability has been been developed. This represents an improvement over the above-mentioned prior art approach of assessment of damage to photosynthesis, which is not as reliable an indicator of the loss of viability after UV-C treatment as the present assay.

From a general perspective, damage to the photosynthesis repair process of the organism is assessed after subjecting the organism to a so-called stressor. As used throughout this specification, the term "stressor" has a broad meaning and is intended to encompass an agent, condition or other stimulus that causes stress to the organism. In a preferred embodiment, the stressor is is selected from the group consisting of: exposure to a chemical, exposure to mechanical energy, thermal shock, dark storage and any combination thereof. In another preferred embodiment, the stressor is ultraviolet radiation such as UV-C radiation.

Thus, in a preferred embodiment, the present invention relates to a protocol of fluorescence measurements during manipulation of the ambient light field after UV-C treatment of an aqueous liquid containing the organism. This preferred embodiment relates to a method for rapid assessment of metabolic impairment in photosynthetic organisms that is significantly more accurate as a measure of loss of viability than methods based on vital stains or on the direct determination of fluorescence parameters alone.

The invention thus relates a rapid and reliable method for assessing the loss of viability in photosynthetic microorganisms that may be advantageously used in the evaualation of disinfection treatments or other stresses placed on such organisms.

BRIEF DESCRIPTION OF THE DRAWINGS

Embodiments of the present invention will be described with reference to the accompanying drawings, wherein like reference numerals denote like parts, and in which:

Figure 1 illustrates a comparison of UV-C-induced mortality (as $\log_{10}$ reduction in viable cell number) in three microalgal cultures, *Thalassiosira weissflogii*, *Heterosigma akashiwo* and *Isochrysis galbana,* estimated by culture-based experiments and by staining with FDA;

Figure 2 illustrates dose-response curves for three microalgal cultures, *Thalassiosira weissflogii*, *Heterosigma akashiwo* and *Isochrysis galbana* and relationships between viability determined from MPN experiments and the variable fluorescence parameter, $F_v$, measured after treatment;

Figure 3 illustrates dose-response curves for three microalgal cultures, *Thalassiosira weissflogii*, *Heterosigma akashiwo* and *Isochrysis galbana* (left) and relationships between viability determined from MPN experiments and a *Photorepair Index* (PRI), measured immediately after treatment (right);

Figure 4 illustrates an example of determination of the input parameters for *PRI* based on sequential incubations at high and low light intensities - $F_v$ is measured on a sample prior to (*Untreated*) and after (*Treated*) treatment with UVR (in each case, a dark-acclimated sample is exposed to high light and a successive period of low light, during which $F_v$ is measured);

Figure 5 illustrates an example of determination of the input parameters for *PRI* based on parallel incubations at high light with and without a chloroplastic protein synthesis inhibitor - in this case, the antibiotic lincomycin was used as an inhibitor ($F_v$ is measured on a sample prior to (*Untreated*) and after (*Treated*) treatment with UVR and, in each case, a dark-acclimated sample is exposed to high light in parallel incubations with and without the protein synthesis inhibitor);

Figure 6 illustrates a schematic of implementation of a first embodiment of the present method; and

Figure 7 illustrates a schematic of implementation of a second embodiment of the present method.

DETAILED DESCRIPTION OF THE PREFERRED EMBODIMENTS

The present disclosure relates to the following independent uses:

use of fluorescence in an assay for loss of viability of an organism in an aqueous liquid after the organism as been exposed to a stressor, the use comprising assessing the ability of the organism to undergo photorepair;

use of fluorescence in an assay for loss of viability of an organism in an aqueous liquid after the organism as been exposed to radiation, the use comprising assessing the ability of the organism to undergo photorepair;

use of fluorescence in an assay for loss of viability of an organism in an aqueous liquid after the organism as been exposed to ultraviolet radiation, the use comprising assessing the ability of the organism to undergo photorepair;

use of variable fluorescence in an assay for loss of viability of an organism in an aqueous liquid after the organism as been exposed to a stressor, the use comprising assessing the ability of the organism to undergo photorepair;

use of variable fluorescence in an assay for loss of viability of an organism in an aqueous liquid after the organism as been exposed to radiation, the use comprising assessing the ability of the organism to undergo photorepair; and

use of variable fluorescence in an assay for loss of viability of an organism in an aqueous liquid after the organism as been exposed to ultraviolet radiation, the use comprising assessing the ability of the organism to undergo photorepair.

Preferred embodiments of these uses may include any one or a combination of any two or more of any of the following features:

- the stressor is selected from the group consisting of: exposure to a chemical, exposure to mechanical energy, thermal shock, dark storage and any combination thereof;
- the radiation is UV-C radiation;
- the ultraviolet radiation has a wavelength in the range of from about 100 nm to about 280 nm;
- the organism is a microorganism;
- the aqueous liquid is water; and/or
- the aqueous liquid is ballast water from a shipping vessel.

The present invention relates to a method for assaying for loss of viability of an organism in an aqueous liquid after the organism has been exposed to a stressor, the method comprising the step of assessing the ability of the organism to undergo photorepair, as defined in claim 1.

Preferred embodiments of these use may include any one or a combination of any two or more of any of the following features:

- the stressor is selected from the group consisting of: exposure to a chemical, exposure to mechanical energy, thermal shock, dark storage and any combination thereof;

- the stressor is ultraviolet radiation;

- the stressor is UV-C radiation;

- the stressor is ultraviolet radiation having a wavelength in the range of from about 100 nm to about 280 nm;

- the assessing step comprises conducting a fluorescence test on the organism;

- the assessing step comprises conducting a variable fluorescence test on the organism;

- the organism is a microorganism;

- the aqueous liquid is water; and/or

- the aqueous liquid is ballast water from a shipping vessel.

In another of its aspects, the present invention relates to a method for assaying for loss of viability of an organism in an aqueous liquid after the organism has been exposed to a stressor, the method comprising the step of correlating the photorepair index for the organism in the aqueous liquid to survivorship of the organism after the organism has been exposed to the stressor. Preferred embodiments of these uses may include any one or a combination of any two or more of any of the following features:

- the stressor is selected from the group consisting of: exposure to a chemical, exposure to mechanical energy, thermal shock, dark storage and any combination thereof;

- the stressor is ultraviolet radiation;

- the stressor is UV-C radiation;

- the stressor is ultraviolet radiation having a wavelength in the range of from about 100 nm to about 280 nm;

- the photorepair index is calculated by subjecting the organism to a fluorescence test;

- the photorepair index is calculated by subjecting the organism to a variable fluorescence test;

- the organism is a microorganism;

- the aqueous liquid is water; and/or

- the aqueous liquid is ballast water from a shipping vessel.

In another of its aspects, the present invention relates to method for assaying for loss of viability of an organism comprised in an aqueous liquid after the organism has been exposed to a stressor, the method comprising the steps of: (a) measuring the variable fluorescence ($F_v$) of an untreated sample of the organism prior to exposure to the stressor; (b) measuring the variable fluorescence ($F_v$) of a treated sample of the organism after exposure to the stressor; (c) calculating a photorepair index using the measurements obtained in Steps (a) and (b); and (d) correlating the photorepair index calculated in Step (c) to a normalized viability for the organism. Preferred embodiments of these use may include any one or a combination of any two or more of any of the following features:

- Step (a) comprises one or more of the following: (i) measuring the variable fluorescence ($F_v$) of the untreated sample of the organism prior to exposure to radiation to obtain $F_v(0)^{Untreated}$; (ii) incubating the untreated sample in the presence of radiation at a first intensity to induce a prescribed reduction in $F_v$ from $F_v(0)^{Untreated}$; (iii) measuring the variable fluorescence ($F_v$) of the untreated sample after Step (ii) to obtain $F_v(1)^{Untreated}$; (iv) incubating the untreated sample in presence of radiation at a second intensity to induce photorepair and an increase in $F_v$ from $F_v(1)^{Untreated}$; and (v) measuring the variable fluorescence of the untreated sample after Step (iv) to obtain $F_v(2)^{Untreated}$;

- Step (ii) is conducted for a period of from about 10 minutes to about 120 minutes;

- the first intensity is in the range of from about 2 $\mu$mol photons m$^{-2}$s$^{-1}$ to about 4000 $\mu$mol photons m$^{-2}$s$^{-1}$;

- Step (ii) comprises incubating the untreated sample in presence of photosynthetically active radiation;

- Step (ii) comprises incubating the untreated sample in presence of photosynthetically active radiation having one or more wavelengths substantially within the range of from about 400 nm to about 700 nm;

- the prescribed reduction in $F_v$ in Step (ii) is in the range of from about 20% to 100%;

- the prescribed reduction in $F_v$ in Step (ii) is in the range of from about 30% to 80%;

- the prescribed reduction in $F_v$ in Step (ii) is in the range of from about 35% to 55%;

- Step (iv) is conducted for a period of from about 30 minutes to about 240 minutes;

- the second intensity is in the range of from about 1 $\mu$mol photons m$^{-2}$s$^{-1}$ to about 100 $\mu$mol photons m$^{-2}$s$^{-1}$;

- the second intensity is in the range of from about 10 $\mu$mol photons m$^{-2}$s$^{-1}$ to about 50 $\mu$mol photons m$^{-2}$s$^{-1}$;

- Step (iv) comprises incubating the untreated sample in presence of photosynthetically active radiation having wavelengths from about 400 nm to about 700 nm;

- Step (iv) comprises incubating the untreated sample in presence of photosynthetically active radiation having one or more wavelengths substantially within the range of from about 400 nm to about 700 nm;

- substantially the same radiation is used Step (ii) and Step (iv);

- Step (b) comprises one or more of the following: (i) incubating the treated sample in presence of photosynthetically active radiation at a first intensity to induce a prescribed reduction in $F_v$ from $F_v(0)^{Untreated}$; (ii) measuring the variable fluorescence of the treated sample after Step (ii) to obtain $F_v(1)^{Treated}$; (iii) incubating the untreated sample in presence of radiation at a second intensity to induce photorepair and an increase in $F_v$ from $F_v(1)^{Treated}$; and (iv) measuring the variable fluorescence of the treated sample after Step (iii) to obtain $F_v(2)^{Treated}$;

- Step (i) is conducted for a period of from about 10 minutes to about 120 minutes;

- the first intensity is in the range of from about 2 $\mu$mol photons m$^{-2}$s$^{-1}$ to about 4000 $\mu$mol photons m$^{-2}$s$^{-1}$;

- Step (i) comprises incubating the untreated sample in presence of photosynthetically active radiation having wavelengths from about 400 nm to about 700 nm;

- Step (i) comprises incubating the untreated sample in presence of photosynthetically active radiation having one or more wavelengths substantially within the range of from about 400 nm to about 700 nm;

- Step (iii) is conducted for a period of from about 30 minutes to about 240 minutes;

- the second intensity is in the range of from about 1 $\mu$mol photons m$^{-2}$s$^{-1}$ to about 100 $\mu$mol photons m$^{-2}$s$^{-1}$;

- the second intensity is in the range of from about 10 $\mu$mol photons m$^{-2}$s$^{-1}$ to about 50 $\mu$mol photons m$^{-2}$s$^{-1}$;

- Step (iii) comprises incubating the untreated sample in presence of photosynthetically active radiation having wavelengths from about 400 nm to about 700 nm;

- Step (iii) comprises incubating the untreated sample in presence of photosynthetically active radiation having one or more wavelengths substantially within the range of from about 400 nm to about 700 nm;

- substantially the same radiation is used Step (i) and Step (iii);

- Step (c) comprises calculating the photorepair index using one of the following equations:

$$PRI = \frac{[F_v(2) - F_v(1)]^{Treated}}{[F_v(0)]^{Untreated}} \qquad (Eq.\ 1)$$

or

$$PRI = \frac{[F_v(2) - F_v(1)]^{Treated}}{[F_v(2) - F_v(1)]^{Untreated}} \qquad (Eq.\ 2)$$

or

$$PRI = \frac{[F_v(2) - F_v(1)]^{Treated}}{F_v(1)^{Untreated}} \qquad (Eq.\ 3);$$

- the organism is a microorganism;

- the aqueous liquid is water; and/or

- the aqueous liquid is ballast water from a shipping vessel.

In one of its aspects, the present disclosure relates to the use of fluorescence, preferably variable fluorescence, more preferably variable chlorophyll fluorescence, in an assay for loss of organism (preferably microorganism) viability in an aqueous liquid.

Damage to photosynthetic systems can be detected rapidly with sensitive assays of variable chlorophyll fluorescence, $F_v$. This can be achieved, for example, using a fluorometer with modulated excitation, including for example, pump-and-probe, PAM, FRRF or FIRe fluorometers (e.g., Genty et al. 1989; Schreiber et al. 1995; Gorbunov and Falkowski 2004). $F_v$ can also be assessed using a fluorometer with a constant excitation when used in conjunction with an electron transport inhibitor such as 3-(3,4-dichlorophenyl)-1,1-dimethylurea (Cullen et al. 1986; Vincent 1980).

Although suggested as means of assessing UV-C-based damage (First and Drake 2013b), $F_v$ alone is not a robust proxy for delayed mortality nor the impairment of reproductive ability in photosynthetic microorganisms. The primary molecular targets for damage to photosynthetic systems are not the same as for UV-C-killing and there is likely to be significant variability between species and growth conditions in the relationship between fluorescence changes and loss of viability (e.g., Campbell et al. 1998; Xiong 2001; Bouchard et al. 2005; Bouchard et al. 2008; Key et al. 2010). The failure of $F_v$ alone to reliably predict UV-C-based mortality is illustrated in Figure 2.

Damage to photosynthetic systems by ultraviolet radiation (UVR) is countered by repair mechanisms. These involve protein synthesis and both mitigate damage during exposure and restore photosynthetic competence after the exposure if the cells are exposed to visible light (Vasilikiotis and Melis 1994; Neidhardt et al. 1998; Adir et al. 2005). The repair mechanisms are thus among the primary targets for UVR-induced damage and mortality; damage to photosynthetic repair mechanisms from UV-C happens at the same time as the more generalized damage to nucleotides that leads to metabolic impairment and the loss of reproductive ability. The present inventors have discovered that measurements of damage to the photosynthesis repair process serve as good proxies for generalized metabolic impairment and the loss of viability.

The present inventors have developed a rapid assay of damage to photosynthetic systems, and repair of that damage, based on measurements of chlorophyll fluorescence. As will be further discussed below, the present inventors have demonstrated that an index based on these measurements can be used to reliably predict survivorship in photosynthetic microorganisms treated with UV-C. In this regard, prior art assessments of damage due to photosynthesis alone were found by the present inventors not to be reliable indicators of the loss of viability after UV-C treatment.

Thus, a protocol of fluorescence measurements during manipulation of the ambient light field after UV-C treatments has been developed by the present inventors. In a preferred embodiment, it is a method for rapid assessment of metabolic impairment in photosynthetic organisms that is significantly more accurate as a measure of loss of viability than methods based on vital stains or on the direct determination of fluorescence parameters alone.

Variable chorophyll a fluorescence, $F_v$ (i.e., maximal fluorescence, $F_m$, minus initial fluorescence, $F_o$) is preferably measured using either a fluorometer with modulated excitation such as a pump-and-probe, pulse amplitude modulated (PAM) fluorometry, fast repetition rate fluorometry (FRRF), fluorescence induction and relaxation (FIRe), etc., or with a fluorometer with a stable excitation intensity in conjunction with an electron transport inhibitor such as 3-(3,4-dichlorophenyl)-1,1-dimethylurea. Data collection protocols are described by the manufacturer of the particular instrument used.

To optimize the accuracy of measurement of $F_v$, it is preferred that each sample be subjected to a period of dark acclimation sufficient to restore photochemical quenching before each measurement of fluorescence.

Preferably, time-dependent changes in $F_v$ are assessed for an untreated sample and for a sample or samples subjected to stress.

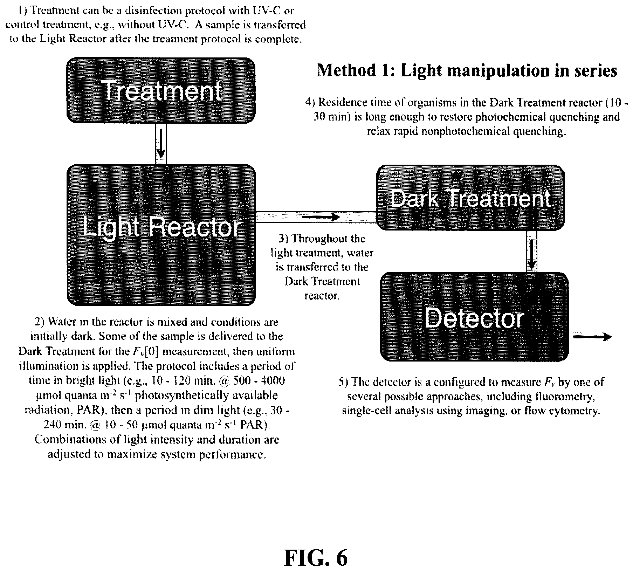

In preferred embodiment, the stress is in the form of exposure to UV-C radiation - this is shown schematically in Figure 6.

Preferably, both the untreated sample and the treated sample(s) are subjected to the same experimental protocol. Preferably, all samples are maintained at temperatures corresponding to conditions in their parent populations (i.e., the temperature in the water body from which they are collected).

Preferably, $F_v$ is measured over the course of an assessment protocol under the following consecutive irradiance conditions.

First, a sample is dark-acclimated and $F_v$ is measured prior to illumination. In the case of the untreated sample, this is $F_v(0)^{Untreated}$.

The sample is then incubated, preferably in visible light, at an irradiance high enough and for a period long enough to induce a prescribed or pre-determined reduction (e.g., 50%) in $F_v$ in the untreated sample. For example, the sample can be exposed for a period of from about 10 minutes to about 120 minutes to photosynthetically active radiation (PAR) having an intensity of from about 2 $\mu$mol photons m$^{-2}$ s$^{-1}$ to about 4000 $\mu$mol photons m$^{-2}$ s$^{-1}$. Preferably, the irradiance is dominated by wavelengths of from about 400 nm to about 700 nm. Alternately, UV-B and UV-A radiation (about 280 nm to 400 nm) can be applied. Non-limiting examples of suitable radiation sources for this purpose may be selected from xenon lamps, quartz halogen lamps, fluorescent lamps, light emitting diodes and the like. As will be further developed below, the value of $F_v$ at the end of the incubation, measured as a single-point value or from an equation fitted to a time-series of measurements, is designated $F_v(1)$.

The sample is then incubated at a lower irradiance that is still high enough to allow for net photorepair and recovery of $F_v$. For example, the sample can be incubated for a period of from about 30 minutes to about 240 minutes to PAR having an intensity of from about 10 $\mu$mol photons m$^{-2}$s$^{-1}$ to about 50 $\mu$mol photons m$^{-2}$s$^{-1}$. Preferably, the irradiance is dominated by wavelengths of from about 400 nm to about 700 nm and can be generated, for example, using a radiation sources as described in the preceding paragraph. The value of $F_v$ at the end of the incubation, measured as a single-point value or from an equation fitted to a time-series of measurements, is designated $F_v(2)$.

The photorepair index (*PRI*) is based on the ratio of $F_v$ in the treated sample to either the intial $F_v$ or the recovered $F_v$ in the untreated sample:

$$PRI = \frac{[F_v(2) - F_v(1)]^{Treated}}{[F_v(0)]^{Untreated}} \qquad \text{(Eq. 1)}$$

or

$$PRI = \frac{[F_v(2) - F_v(1)]^{Treated}}{[F_v(2) - F_v(1)]^{Untreated}} \qquad \text{(Eq. 2)}$$

or

$$PRI = \frac{[F_v(2) - F_v(1)]^{Treated}}{F_v(1)^{Untreated}}$$ (Eq. 3).

where $F_v(1)$ and $F_v(2)$ are defined as described above, *Untreated* refers to control samples that are not exposed to treatments such as UVR, *Treated* refers to the sample post UV treatment, and $F_v(0)$ refers to the sample prior to any treatment.

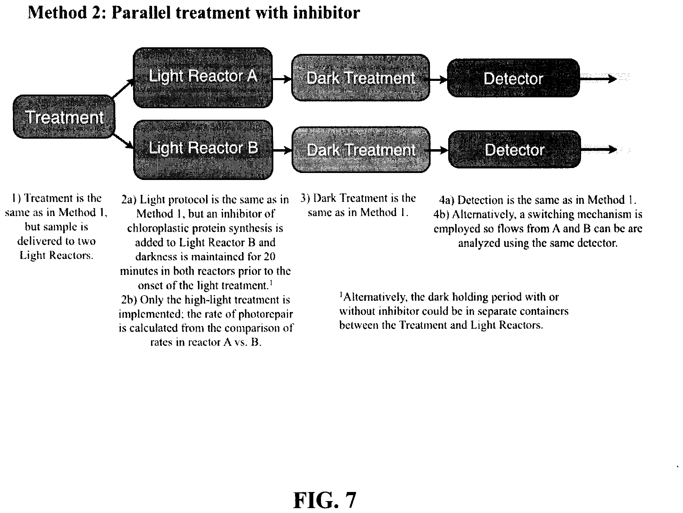

In an alternate embodiment of the invention, $F_v$ may also be measured during parallel incubations treated with and without a chloroplastic protein synthesis inhibitor (e.g., antibiotics such as streptomycin, lincomycin, azithromycin, etc.) and thereby incable of photorepair; the results can be used to validate interpretions of damage versus repair - this is shown schematically in Figure 7.

When determined with a protein synthesis inhibitor, it is again preferred that both the *Untreated* and UVR-treated (*Treated*) samples are subjected to the same assay protocol. Preferably, the protocol is as follows.

The sample is mixed and divided into two aliquots. One aliquot is treated with an appropriate dose of a protein synthesis inhibitor (e.g., 200-1000 g l$^{-1}$ lincomycin).

The untreated sample is a control. Both samples are incubated in darkness at assay temperature for a period of at least about 10 minutes, more preferably at least about 15 minutes, most preferably 20 minutes.

$F_{v,Initial}$ is measured on both untreated control and antibiotic-treated sub-samples prior to illumination.

Both sub-samples are then incubated at an irradiance high enough and for a period long enough to induce a prescribed or pre-determined reduction (e.g., 50%) in the untreated sub-sample - for example, using the above-described time periods and radiation intensities.

The photorepair index (*PRI*) is based on the difference in rates of decline of $F_v$ between the control and protein synthesis inhibitor-treated sub-samples. Preferably, the rates of decline are characterized by fitting to a first-order model, for example:

$$F_{v,t} = F_{v,Initial} \bullet \exp(-kt)$$ (Eq. 4)

where $F_{v,t}$ is $F_v$ at time $t$ during high-light exposure, $F_{v,Initial}$ is $F_v$ prior to high-light exposure, and $k$ is a first-order rate constant that is evaluated for both the inhibited and uninhibited sub-samples ($k_{Inhibited}$, $k_{Control}$). Eq. 4 can be modified to include $F_{v,\infty}$, a non-zero asymptotic value of $F_v$ :

$$F_{v,t} = F_{v,Initial} \bullet \exp(-kt) + F_{v,\infty}$$ (Eq. 5)

In either case, *PRI* is calculated as a function of the difference between inhibited and uninhibited rate constants for the *Treated* (e.g., with UVR) subsample vs. the *Untreated* sample:

$$PRI = \frac{\left[ F_{v,Initial} \bullet (k_{Inhibited} - k_{Control}) \right]^{Treated}}{\left[ F_{v,Initial} \bullet (k_{Inhibited} - k_{Control}) \right]^{Untreated}}$$ (Eq. 6)

where $k_{Inhibited}$ and $k_{Control}$ are the rate constants for the sub-sample treated with the protein synthesis inhibitor and the control sub-sample, respectively, and where *Treated* refers to the sample post-UVR treatment, and *Untreated* refers to the sample prior to any treatment.

The photorepair index is related to the probability of survivorship as determined through experiments on microorganisms subjected to UVC and assayed for both *PRI* and the reduction in viability as determined through culture-based experiments (e.g., Most Probable Numbers, MPN, Fig. 1-3).

Preferred embodiments of the present application will be illustrated with reference to the following examples, which are not intended to construe or limit the scope of the present invention:

Example 1

Cultures of marine microalgae were used in the Examples. These were obtained from the Provasoli-Guillard National Center for Marine Algae (East Boothbay, ME, USA) and maintained at low optical density at 18°C on a 12:12 L:D cycle.

Illumination was provided by cool-white fluorescent bulbs at an intensity of 80 $\mu$mol photons m$^{-2}$s$^{-1}$ PAR.

**[0053]** Cultures were maintained in nutrient-replete balanced growth at constant density by daily dilution with fresh medium (MacIntyre and Cullen 2005). Cultures were monitored daily for dark-acclimated chlorophyll *a* fluorescence (Brand et al., 1981) using a 10-AU fluorometer (Turner Designs, San Jose, CA, USA) and a FIRe fluorometer (Satlantic, Halifax, NS, Canada). Both fluorometers were blanked daily and fluorescence was normalized to a 200 $\mu$M rhodamine standard.

Daily specific growth rates ($\mu$, d$^{-1}$) were calculated from the dilution-corrected change in fluorescence over the preceding 24 h assuming exponential growth (MacIntyre and Cullen 2005). The single-turnover fluorescence induction curve measured with the FIRe was fitted using the MATLAB routine *Fireworx 1.0.4* (Barnett) to estimate minimum and maximum fluorescence ($F_0$ and $F_m$, Arb.), variable fluorescence ($F_v = F_m - F_0$, Arb.), and the quantum yield of Photosystem II electron transport ($F_v/F_m$, dimensionless). Cultures were considered to be in balanced growth when the coefficients of variation (C.V.) for $\mu$ and $F_v/F_m$ were <10% for a minimum of 10 generations.

Cultures in balanced growth were subjected to defined doses of UV-C radiation delivered by a conventional low-pressure collimated beam source (Bolton and Linden 2003). Cultures were irradiated in 50-ml aliquots in a reaction vessel (50 mm diameter, 25 mm depth) centered under the UV beam. Cultures were stirred (approx. 60 r.p.m.) with a miniature magnetic stir-bar during dosage to ensure homogenous application of the dose.

The intensity of the UV beam was measured with a NST-traceable ILT1700 radiometer (International Light Technologies, Peabody, MA, USA). Homogeneity of exposure over the surface of the reaction vessel was verified as being <5% (C.V.) by measuring beam intensity at 5-mm intervals over perpendicular axes aligned with the center of the reaction vessel. Beam attenuation through the culture at 254 nm was measured with a UV254 Series 'P' meter RealTech, Whitby, ON). The mean dosage in the reaction vessel was calculated from the incident intensity and the attenuation at 254 nm, by application of the Lambert-Beer law. The dosage (mJ cm$^{-2}$) was then set by calculating the appropriate duration of exposure, given that dosage is the product of the mean intensity in the reaction vessel (mW cm$^{-2}$) and the duration of exposure (s).

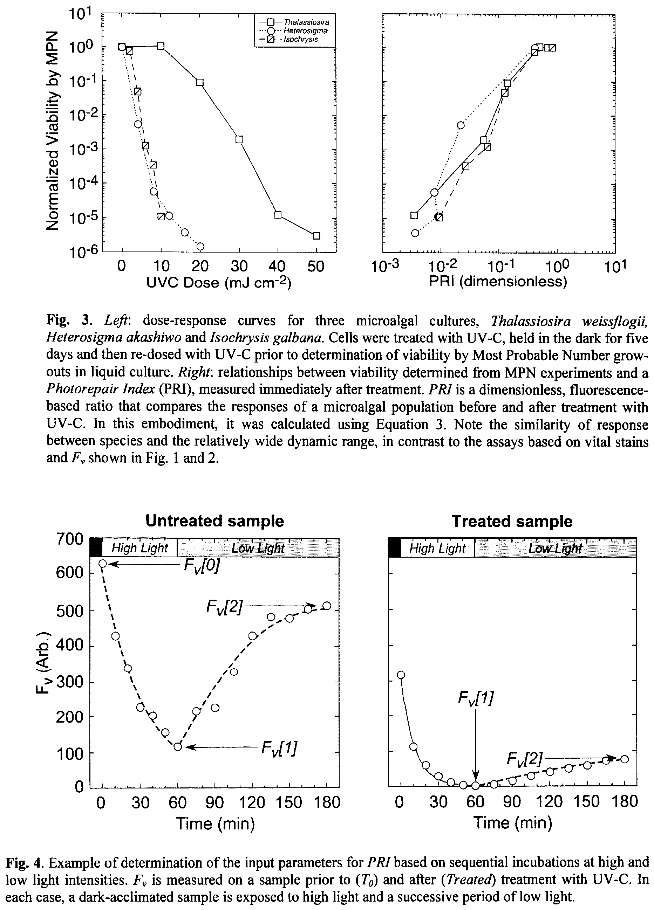

A photorepair index was calculated from the recovery of $F_v$ at low irradiance following application of a photoinhibitory PAR light regime - see Figure 4. An exponentially-growing culture was divided into two aliquots. One, the *Untreated,* sample, was assayed immediately; the second *Treated* sample was irradiated with UV-C before assay.

The two samples were otherwise subjected to identical assay conditions. Each was dark-acclimated for a minimum of 20 minutes to allow photochemical quenching to be restored and short-lived fluorescence quenching to relax. A subsample was taken at the end of this period and $F_v$ was measured using the FIRe fluorometer. The remainder of the sample was then incubated at an irradiance of 550 $\mu$mol photons m$^{-2}$s$^{-1}$ of photosynthetically active radiation (PAR, 400 nm - 700 nm) for 60 minutes.

The sample was held at growth temperature in a water-cooled manifold illuminated by a programmable warm-white LED array (Photon Systems International, Brno, Czech Republic). Sub-samples were removed at 10-min intervals and dark-acclimated for a minimum of 20 minutes prior to determination of $F_v$. These are designated as the "High-Light" samples in Figure 4.

Following the high-light exposure, the remaining sample was incubated at growth temperature at an irradiance of 20 $\mu$mol photons m$^{-2}$ s$^{-1}$ of PAR by the same LED array. Sub-samples were removed at 15-minute intervals and dark-acclimated for a minimum of 20 minutes for determination of $F_v$. These are designated as the "Low-Light" samples in Figure 4.

The input parameters for permutations of the PRI were derived by fitting the kinetic variations in $F_v$ over time in the two different regimes and for each of the *Untreated* and *Treated* samples and the results are shown in Figure 4 (results are shown in Figure 3 for several different treatments).

Example 2

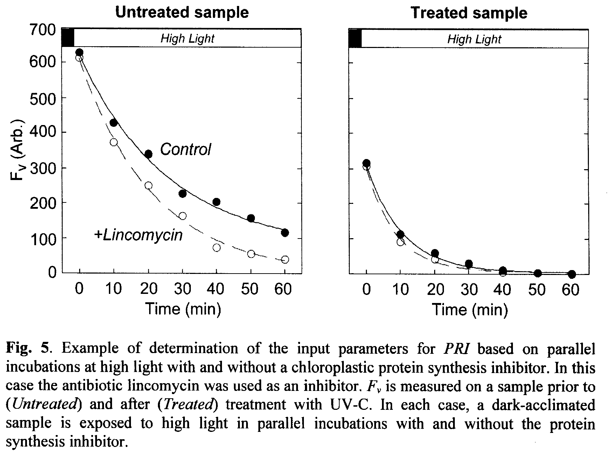

In this example, the photorepair index was calculated from the differential loss of $F_v$ during application of a photoinhibitory light regime with and without the antibiotic lincomycin, an inhibitor of chloroplastic protein synthesis - see Figure 5.

An exponentially-growing culture was divided into two aliquots. One, the *Untreated* sample, was assayed immediately; the second *Treated* sample was irradiated with UV-C before assay.

Both the *Untreated* and the *Treated* samples were then subdivided into control and antibiotic-treated subsamples. The control samples were assayed without further amendment. The antibiotic-treated samples were treated with an aqueous solution of lincomycin to a final concentration of 500 $\mu$g ml$^{-1}$ and incubated at growth temperature in the dark for 10 minutes to allow uptake of the antibiotic.

Subsequently, all four samples were subjected to identical assay conditions. Each was first dark-acclimated for a minimum of 20 min to allow short-lived fluorescence quenching to relax. A subsample was taken at the end of this period and $F_v$ was measured using the FIRe fluorometer. The remainder of the sample was then incubated at an irradiance of 550 $\mu$mol photons m$^{-2}$ s$^{-1}$ of Photosynthetically Active Radiation (PAR, 400-700 nm) for 60 minutes. The sample was held at growth temperature in a water-cooled manifold illuminated by a programmable warm-white LED array (Photon Systems International, Brno, Czech Republic). Sub-samples were removed at 10-min intervals and dark-acclimated for a minimum of 20 min for determination of $F_v$. These are designated as the "High-Light" samples in Fig. 5.

The input parameters for permutations of the PRI were derived by fitting the kinetic variations in $F_v$ over time in both the control and antibiotic-treated subsamples of each of the *Untreated* and *Treated* samples.

Example 3

Viability of the cultures subsequent to treatment with UV-C was assessed by the Most Probable Number (MPN) assay (Cochran 1950; Blodgett 2005a,b).

Thus, the cultures were diluted in 3 log-interval series (e.g. 10$^{-1}$, 10$^{-2}$ and 10$^{-3}$) with fresh growth medium. The appropriate dilution range for any UV-C dose was determined in preliminary, range-finding experiments.

For each culture, five replicates of each dilution were then incubated at an irradiance and temperature optimal for growth (typically 160 $\mu$mol photons m$^{-2}$ s$^{-1}$ of PAR and 22 °C) and monitored for growth every 48 h using the 10-AU fluorometer. Tubes were scored positive for growth if fluorescence increased by an order of magnitude above the limit of quantitation (Anderson 1989) or the initial fluorescence reading, whichever was higher.

The Most Probable Number of viable cells was then obtained from look-up tables (Blodgett 2010) and converted to a concentration from the volume of culture in each tube and the range of dilutions used. The concentrations of viable cells obtained by the MPN analyses were used to construct dose-response curves for UV exposure and for comparison with the PRI - see Figure 3. As can be seen in Figure 3, the similarity of response between species and the relatively wide dynamic range is superior to the assays based on vital-stain and $F_v$ shown in Figures 1 and 2.

While this invention has been described with reference to illustrative embodiments and examples, the description is not intended to be construed in a limiting sense. Thus, various modifications of the illustrative embodiments, as well as other embodiments of the invention, will be apparent to persons skilled in the art upon reference to this description. For example, while a preferred embodiment of the present invention relates to the use of fluorescence in an assay for loss of organism (preferably microorganism) viability in an aqueous liquid, it is possible to adapt this preferred embodiment to the use of fluorescence in an assay for loss of organism (preferably microorganism) viability in other than an aqueous liquid - e.g., organisms (preferably microorganisms) that have been isolated on a filter or otherwise removed from the medium in which they typically exist. Thus, it is possible to modify the schematic illustrated in Figure 6 to include filter element or other organism (preferably microorganism) isolating element prior to the Dark Treatment or Detector elements. It is therefore contemplated that the appended claims will cover any such modifications or embodiments.

LIST OF DOCUMENTS CITED IN SPECIFICATION

(1) Adir, N., H. Zer, S. Shochat and I. Ohad (2005). Photoinhibition - a historical perspective. Discoveries in Photosynthesis. Govindjee, J.T. Beatty, H. Gest and J.F. Allen. Dordrecht, The Netherlands, Springer: 931-958.

(2) Agustí, S. and M.C. Sanchez (2002). Cell viability in natural phytoplankton communities quantified by a membrane permeability probe. Limnol. Oceanogr. 47(3): 818-828.

(3) Anderson, D. J. (1989). "Determination of the lower limit of detection." Clin. Chem. 35(10): 2152-2153.

(4) Blodgett, R. J. (2005a). "Serial dilution with a confirmation step." Food Microbiol. 22(6): 547-552.

(5) Blodgett, R. J. (2005b). "Upper and lower bounds for a serial dilution test." J. AOAC Int. 88(4): 1227-1230.

(6) Blodgett, R. J. (2010). Appendix 2: Most Probable Number from Serial Dilutions. Bacteriological Analytical Manual, US Food and Drug Administration.

(7) Bolton, J. R. and K. G. Linden (2003). "Standardization of methods for fluence (UV dose) determination in bench-scale UV experiments." J. Environ. Eng.-ASCE 129(3): 209-215.

(8) Bouchard, J.N., D.A. Campbell and S. Roy (2005). Effects of UV-B radiation on the D1 protein repair cycle of natural phytoplankton communities from three latitudes (Canada, Brazil, and Argentina). J. Phycol. 41: 273-286.

(9) Brand, L. E., R. R. L. Guillard, and L.S. Murphy (1981). "A method for the rapid and precise determination of acclimated phytoplankton reproduction rates." J. Plankton Res. 3: 193-201.

(10) Bouchard, J.N., M.L. Longhi, S. Roy, D.A. Campbell and G. Ferreyra (2008). Interaction of nitrogen status and UVB sensitivity in a temperate phytoplankton assemblage. J. Exp. Mar. Biol. Ecol. 359: 67-76.

(11) Campbell, D., V. Hurry, A.K. Clarke, P. Gustafsson and G. Öquist (1998). Chlorophyll fluorescence analysis of cyanobacterial photosynthesis and acclimation. Microbiology and Molecular Biology Reviews 62: 667-683.

(12) Cochran, W.G. (1950). "Estimation of bacterial densities by means of the most probable number." Biometrics 6(2): 105-116.

(13) Cullen, J.J., M. Zhu and D.C. Pierson (1986). A technique to assess the harmful effects of sampling and containment for determination of primary production. Limnol. Oceanogr. 31: 1364-1373.

(14) Dorsey, J., C.M. Yentsch, S. Mayo and C. McKenna (1989). Rapid analytical technique for the assessment of cell metabolic activity in marine microalgae. Cytometry 10: 622-628.

(15) ETV (2010). Generic protocol for the verification of ballast water treatment technology. Program, NSF International for USEPA Environmental Technology Verification Program, Ann Arbor, MI.

(16) Gorbunov, M.Y. and P.G. Falkowski. Fluorescence induction and relaxation (FIRe) technique and instrumentation for monitoring photosynthetic processes and primary production in aquatic ecosystems. Fundamental Aspects to Global Perspectives, Bruce, D., and A. van der Est, Eds. Allen Press, Montreal. P. 1029-1031.

(17) First, M.R. and L.A. Drake (2013a). Approaches for determining the effects of UV radiation on microorganisms in ballast water. Management of Biological Invasions 4: 87-99.

(18) First, M.R. and L.A. Drake (2013b). Life after treatment: detecting living microorganisms following exposure to UV light and chlorine dioxide. J Appl Phycol DOI 10.1007/s10811-013-0049-9.

(19) Garvey, M., B. Moriceau and U. Passow (2007). Applicability of the FDA assay to determine the viability of marine phytoplankton under different environmental conditions. Marine Ecology-Progress Series 352: 17-26.

(20) Genty, B., J.-M. Briantais and N.R. Baker (1989). The relationship between the quantum yield of photosynthetic electron transport and quenching of chlorophyll fluorescence. Biochem. Biophys. Acta 990: 87-92.

(21) Gieskes, W.W.C. and A.G.J. Buma (1997). UV damage to plant life in a photobiologically dynamic environment: The case of marine phytoplankton. Plant Ecol. 128(1-2): 16-25.

(22) Gilbert, F., F. Galgani and Y. Cadiou (1992). Rapid assessment of metabolic-activity in marine microalgae - application in ecotoxicological tests and evaluation of water-quality. Mar. Biol. 112(2): 199-205.

(23) Key, T., A. McCarthy, D.A. Campbell, C. Six, S. Roy and Z.V. Finkel (2010). Cell size trade-offs govern light exploitation strategies in marine phytoplankton. Environmental Microbiology 12: 95-104.

(24) Liebich, V., P.P. Stehouwer and M. Veldhuis (2012). Re-growth of potential invasive phytoplankton following UV-based ballast water treatment. Aquat. Invasions 7(1): 29-36.

(25) MacIntyre, H. L. and J. J. Cullen (2005). Using cultures to investigate the physiological ecology of microalgae. Algal Culture Techniques. R. A. Andersen. New York, Academic Press: 287-326.

(26) Murphy, A. and T. Cowles (1997). Effects of darkness on multi-excitation in vivo fluorescence and survival in a marine diatom. Limnol. Oceanogr. 42: 1444-1453.

(27) Neidhardt, J., J.R. Benemann, L. Zhang and A. Melis (1998). Photosystem-II repair and chloroplast recovery from irradiance stress: relationship between chronic photoinhibition, light-harvesting chlorophyll antenna size and photosynthetic productivity in Dunaliella salina (green algae). Photosynth. Res. 56: 175-184.

(28) Onji, M., T. Sawabe and Y. Ezura (2000). An evaluation of viable staining dyes suitable for marine phytoplankton. Bull Fac Fish Hokkaido Univ 51: 151-158.

(29) Peperzak, L. and C.P.D. Brussaard (2011). Flow cytometric applicability of fluorescent vitality probes on phytoplankton. J. Phycol. 47(3): 692-702.

(30) Schreiber, U., H. Hormann, C. Neubauer and C. Klughammer (1995). Assessment of photosystem II photochemical quantum yield by chlorophyll fluorescence quenching analysis. Aust. J. Plant Physiol. 22: 209-220.

(31) Selvin, R., B. Requera, I. Bravo and C.M. Yentsch (1988). Use of fluorescein diacetate (FDA) as a single-cell probe of metabolic activity in dinoflagellate cultures. Bio. Oceanogr. 6: 505-511.

(32) Sinha, R.P. and D.-P. Hader (2002). UV-induced DNA damage and repair: a review. Photochemical & Photobiological Sciences 1(4): 225-236.

(33) Steinberg, M.K., E.J. Lemieux and L.A. Drake (2011). Determining the viability of marine protists using a combination of vital, fluorescent stains. Mar. Biol. 158: 1431-1437.

(34) Throndsen, J. (1978), The dilution-culture method, in Phytoplankton Manual, edited by A. Sournia, pp. 218-224, UNESCO, Paris.

(35) Vasilikiotis, C. and A. Melis (1994). Photosystem II reaction center damage and repair cycle: chloroplast acclimation strategy to irradiance stress. Proc. Nat. Acad. Sci 91: 7222-7226.

(36) Vincent, W.F. (1980). Mechanisms of rapid photosynthetic adaptation in natural phytoplankton communities. II. Changes in photochemical capacity as measured by DCMU-induced chlorophyll fluorescence. J. Phycol. 16: 568-577.

(37) Xiong, F. (2001). Evidence that UV-B tolerance of the photosynthetic apparatus in microalgae is related to the D1-turnover mediated repair cycle in vivo. J. Plant Phys. 158: 285-294.

**Claims**

1. A method for assaying for loss of viability of a microorganism comprised in water after the microorganism has been treated by exposure to a stressor, said stressor being an agent, condition or stimulus that causes stress to the microorganism, the method aiming to quantify both damage to photosystems and their repair, the method comprising the steps of:

   a) measuring the variable fluorescence ($F_v$) of an untreated sample of the microorganism;
   b) measuring the variable fluorescence ($F_v$) of a treated sample of the microorganism;
   c) calculating a photosynthesis repair process index using the measurements obtained in Steps (a) and (b), the photorepair index involving protein synthesis; and
   d) correlating the photosynthesis repair process index calculated in Step (c) to a normalized viability for the microorganism, wherein Step (a) comprises one or more of the following:

      (i) measuring the variable fluorescence ($F_v$) of the untreated sample of the microorganism to obtain a first value ($F_v(0)^{Untreated}$);
      (ii) incubating the untreated sample in the presence of radiation at a first intensity to induce a prescribed reduction in variable fluorescence ($F_v$) from said first value ($F_v(0)^{Untreated}$);
      (iii) measuring the variable fluorescence ($F_v$) of the untreated sample after Step (ii) to obtain a second value ($F_v(1)^{Untreated}$);

(iv) incubating the untreated sample in presence of radiation at a second intensity to induce a photosynthesis repair process involving protein synthesis and an increase in variable fluorescence ($F_v$) from said second value ($F_v(1)^{Untreated}$); and

(v) measuring the variable fluorescence of the untreated sample after Step (iv) to obtain a third value ($F_v(2)^{Untreated}$).

2. The method defined in any one of Claim 1, wherein the prescribed reduction in variable fluorescence ($F_v$) in Step (ii) is in the range of from about 30% to 80%.

3. The method defined in any one of Claims 1-2, wherein Step (iv) is conducted for a period of from about 30 minutes to about 240 minutes.

4. The method defined in any one of Claims 1-3, wherein the second intensity is in the range of from about 1 $\mu$mol photons m$^{-2}$ s$^{-1}$ to about 50 $\mu$mol photons m$^{-2}$s$^{-1}$.

5. The method defined in any one of Claims 1-4, wherein Step (iv) comprises incubating the untreated sample in presence of photosynthetically active radiation having one or more wavelengths substantially within the range of from about 400 nm to about 700 nm.

6. The method defined in any one of Claims 1-5, wherein Step (b) comprises one or more of the following:

(i) incubating the treated sample in presence of photosynthetically active radiation at a first intensity to induce a prescribed reduction in variable fluorescence ($F_v$) from said first value ($F_v(0)^{Treated}$);

(ii) measuring the variable fluorescence of the treated sample after Step (ii) to obtain said second value ($F_v(1)^{Treated}$);

(iii) incubating the treated sample in presence of radiation at a second intensity to induce a photosynthesis repair process involving protein synthesis and an increase in variable fluorescence ($F_v$) from said second value ($F_v(1)^{Treated}$); and

(iv) measuring the variable fluorescence of the treated sample after Step (iii) to obtain said third value ($F_v(2)^{Treated}$).

7. The method defined in Claim 6, wherein Step (i) comprises incubating the untreated sample in presence of photosynthetically active radiation having one or more wavelengths substantially within the range of from about 400 nm to about 700 nm.

8. The method defined in any one of Claims 6-7, wherein substantially the same radiation is used Step (i) and Step (iii).

9. The method defined in any one of Claims 1-8, wherein Step (c) comprises calculating the photosynthesis repair process index using one of the following equations:

1.

$$PRI = \frac{[F_v(2) - F_v(1)]^{Treated}}{[F_v(0)]^{Untreated}} \quad \text{(Eq. 1)}$$

or

2.

$$PRI = \frac{[F_v(2) - F_v(1)]^{Treated}}{[F_v(2) - F_v(1)]^{Untreated}} \quad \text{(Eq. 2)}$$

or

3.

$$PRI = \frac{[F_v(2) - F_v(1)]^{Treated}}{F_v(1)^{Untreated}}$$

(Eq. 3).

**Patentansprüche**

1. Verfahren zur Untersuchung auf Verlust der Lebensfähigkeit eines Mikroorganismus, der in Wasser enthalten ist, nachdem der Mikroorganismus durch Aussetzen an einen Stressor behandelt wurde, wobei der Stressor ein Mittel, Zustand oder Stimulus ist, der Stress für den Mikroorganismus verursacht, wobei das Verfahren auf die Quantifizierung sowohl von Schäden an Photosystemen als auch ihrer Reparatur abzielt, wobei das Verfahren die Schritte umfasst:

   a) Messen der variablen Fluoreszenz ($F_v$) einer unbehandelten Probe des Mikroorganismus;
   b) Messen der variablen Fluoreszenz ($F_v$) einer behandelten Probe des Mikroorganismus;
   c) Berechnen eines Photosynthese-Reparaturprozessindexes unter Verwendung der in Schritt (a) und (b) erhaltenen Messungen, wobei der der Photoreparaturindex eine Proteinsynthese involviert; und
   d) Korrelieren des Photosynthese-Reparaturprozessindexes, der in Schritt (c) berechnet wird, mit einer normalisierten Lebensfähigkeit für den Mikroorganismus, wobei Schritt (a) einen oder mehrere der folgenden umfasst:

   (i) Messen der variablen Fluoreszenz ($F_v$) der unbehandelten Probe des Mikroorganismus, um einen ersten Wert ($F_v(0)^{Untreated}$) zu erhalten;
   (ii) Inkubieren der unbehandelten Probe in Anwesenheit einer Strahlung mit einer ersten Intensität, um eine vorgeschriebene Reduktion in der variablen Fluoreszenz ($F_v$) von dem ersten Wert ($F_v(0)^{Untreated}$) zu erhalten;
   (iii) Messen der variablen Fluoreszenz ($F_v$) der unbehandelten Probe nach Schritt (ii), um einen zweiten Wert ($F_v(1)^{Untreated}$) zu erhalten;
   (iv) Inkubieren der unbehandelten Probe in Anwesenheit einer Strahlung mit einer zweiten Intensität, um einen Photosynthese-Reparaturprozess zu induzieren, der eine Proteinsynthese und eine Erhöhung der variablen Fluoreszenz ($F_v$) von dem zweiten Wert ($F_v(1)^{Untreated}$) involviert; und
   (v) Messen der variablen Fluoreszenz der unbehandelten Probe nach Schritt (iv), um einen dritten Wert ($F_v(2)^{Untreated}$) zu erhalten.

2. Verfahren nach einem der Ansprüche 1, wobei die vorgeschriebene Reduktion in der variablen Fluoreszenz ($F_v$) in Schritt (ii) im Bereich von ungefähr 30 % bis 80 % liegt.

3. Verfahren nach einem der Ansprüche 1 bis 2, wobei Schritt (iv) für eine Periode von ungefähr 30 Minuten bis ungefähr 240 Minuten durchgeführt wird.

4. Verfahren nach einem der Ansprüche 1 bis 3, wobei die zweite Intensität im Bereich von ungefähr 1 $\mu$mol Photonen m$^{-2}$s$^{-1}$ bis ungefähr 50 $\mu$mol Photonen m$^{-2}$s$^{-1}$ liegt.

5. Verfahren nach einem der Ansprüche 1 bis 4, wobei Schritt (iv) das Inkubieren der unbehandelten Probe in Anwesenheit einer photosynthetisch aktiven Strahlung mit einer oder mehreren Wellenlängen im Wesentlichen innerhalb des Bereichs von ungefähr 400 nm bis ungefähr 700 nm umfasst.

6. Verfahren nach einem der Ansprüche 1 bis 5, wobei Schritt (b) einen oder mehrere der folgenden umfasst:

   (i) Inkubieren der behandelten Probe in Anwesenheit einer photosynthetisch aktiven Strahlung mit einer ersten Intensität, um eine vorgeschriebene Reduktion in der variablen Fluoreszenz ($F_v$) von dem ersten Wert ($F_v(0)^{Treated}$) zu erhalten;
   (ii) Messen der variablen Fluoreszenz ($F_v$) der behandelten Probe nach Schritt (ii), um den zweiten Wert ($F_v(1)^{Treated}$) zu erhalten;
   (iii) Inkubieren der behandelten Probe in Anwesenheit einer Strahlung mit einer zweiten Intensität, um einen Photosynthese-Reparaturprozess zu induzieren, der eine Proteinsynthese und eine Erhöhung der variablen Fluoreszenz ($F_v$) von dem zweiten Wert (($F_v(1)$)^{Treated}) involviert; und

(iv) Messen der variablen Fluoreszenz der behandelten Probe nach Schritt (iii), um den dritten Wert (($F_v(2)$)^{Treated}) zu erhalten.

**7.** Verfahren nach Anspruch 6, wobei Schritt (i) das Inkubieren der unbehandelten Probe in Anwesenheit einer photosynthetisch aktiven Strahlung mit einer oder mehreren Wellenlängen im Wesentlichen innerhalb des Bereichs von ungefähr 400 nm bis ungefähr 700 nm umfasst.

**8.** Verfahren nach einem der Ansprüche 6 bis 7, wobei im Wesentlichen dieselbe Strahlung in Schritt (i) und Schritt (iii) verwendet wird.

**9.** Verfahren nach einem der Ansprüche 1 bis 8, wobei Schritt (c) das Berechnen des Photosynthese-Reparaturprozessindexes unter Verwendung einer der folgenden Gleichungen umfasst:

1.

$$PRI = \frac{\left[F_v(2) - F_v(1)\right]^{Treated}}{\left[F_v(0)\right]^{Untreated}} \qquad (Gl.1)$$

oder
2.

$$PRI = \frac{\left[F_v(2) - F_v(1)\right]^{Treated}}{\left[F_v(2) - F_v(1)\right]^{Untreated}} \qquad (Gl.2)$$

oder
3.

$$PRI = \frac{\left[F_v(2) - F_v(1)\right]^{Treated}}{F_v(1)^{Untreated}} \qquad (Gl.3).$$

**Revendications**

**1.** Procédé pour évaluer la perte de viabilité d'un micro-organisme compris dans de l'eau après le traitement du microorganisme par exposition à un élément stressant, ledit élément stressant est en un agent, une condition ou un stimulus entraînant du stress pour le micro-organisme, le procédé visant à quantifier à la fois les dégâts sur les photosystèmes et leur réparation, ce procédé comprenant les étapes :

a) la mesure de la fluorescence variable ($F_v$) d'un échantillon non traité du micro-organisme ;
b) la mesure de la fluorescence variable ($F_v$) d'un échantillon traité du micro-organisme ;
c) le calcul d'un indice de processus de réparation photosynthétique en utilisant les mesures obtenues aux étapes (a) et (b), l'indice de photoréparation impliquant la synthèse de protéines ; et
d) la corrélation de l'indice de processus de réparation photosynthétique calculé à l'étape (c) à une viabilité normalisée pour le micro-organisme, dans lequel l'étape (a) comprend une ou plusieurs des étapes suivantes :

(i) la mesure de la fluorescence variable ($F_v$) de l'échantillon non traité du micro-organisme pour obtenir une première valeur (($F_v(0)$)^{non traité}) ;

(ii) l'incubation de l'échantillon non traité en présence d'un rayonnement à une première intensité pour induire une réduction prescrite de la fluorescence variable ($F_v$) à partir de ladite première valeur ($F_v(0)^{non\ traité}$) ;

(iii) la mesure de la fluorescence variable ($F_v$) de l'échantillon non traité après l'étape (ii) pour obtenir une deuxième valeur ($F_v(1)^{non\ traité}$) ;

(iv) l'incubation de l'échantillon non traité en présence d'un rayonnement à une deuxième intensité pour induire un processus de réparation photosynthétique impliquant la synthèse de protéines et une augmentation de la fluorescence variable ($F_v$) à partir de ladite deuxième valeur ($Fv(1)^{non\ traité}$) ; et

(v) la mesure de la fluorescence variable de l'échantillon non traité après l'étape (iv) pour obtenir une troisième valeur ($F_v(2)^{non\ traité}$).

2. Procédé défini à la revendication 1, dans lequel la réduction prescrite de la fluorescence variable valeur ($F_v$) à l'étape (ii) est comprise entre environ 30 % et 80 %.

3. Procédé défini à l'une quelconque des revendications 1 et 2, dans lequel l'étape (iv) est mise en oeuvre pendant une période comprise entre environ 30 minutes et environ 240 minutes.

4. Procédé défini à l'une quelconque des revendications 1 à 3, dans lequel la deuxième intensité est comprise entre environ 1 $\mu$mol photons m$^{-2}$s$^{-1}$ et environ 50 $\mu$mol photons m$^{-2}$s$^{-1}$.

5. Procédé défini à l'une quelconque des revendications 1 à 4, dans lequel l'étape (iv) comprend l'incubation de l'échantillon non traité en présence d'un rayonnement photosynthétiquement actif présentant une ou plusieurs longueurs d'onde sensiblement comprises entre environ 400 nm et environ 700 nm.

6. Procédé défini à l'une quelconque des revendications 1 à 5, dans lequel l'étape (b) comprend une ou plusieurs des étapes suivantes :

(i) l'incubation de l'échantillon traité en présence d'un rayonnement photosynthétiquement actif à une première intensité pour induire une réduction prescrite de la fluorescence variable ($F_v$) à partir de ladite première valeur ($F_v(0)^{traité}$) ;

(ii) la mesure de la fluorescence variable de l'échantillon traité après l'étape (ii) pour obtenir ladite deuxième valeur ($F_v(1)^{traité}$) ;

(iii) l'incubation de l'échantillon traité en présence d'un rayonnement à une deuxième intensité pour induire un processus de réparation photosynthétique impliquant la synthèse de protéines et une augmentation de la fluorescence variable ($F_v$) à partir de ladite deuxième valeur ($F_v(1)^{traité}$) ; et

(iv) la mesure de la fluorescence variable de l'échantillon traité après l'étape (iii) pour obtenir ladite troisième valeur ($F_v(2)^{traité}$).

7. Procédé défini à la revendication 6, dans lequel l'étape (i) comprend l'incubation de l'échantillon non traité en présence d'un rayonnement photosynthétiquement actif présentant une ou plusieurs longueurs d'onde sensiblement comprises entre environ 400 nm et environ 700 nm.

8. Procédé défini à l'une quelconque des revendications 6 et 7, dans lequel on utilise sensiblement le même rayonnement à l'étape (i) et à l'étape (iii).

9. Procédé défini à l'une quelconque des revendications 1 à 8, dans lequel l'étape (c) comprend le calcul de l'indice de processus de réparation photosynthétique en utilisant l'une des équations suivantes :

1.

$$PRI = \frac{[F_v(2) - F_v(1)]^{traité}}{[F_v(0)]^{non\ traité}} \qquad (\text{Eq. 1})$$

2.

$$PRI = \frac{[F_v(2)-F_v(1)]^{traité}}{[F_v(2)-F_v(1)]^{non\ traité}} \quad (Eq.\ 2)$$

3.

$$PRI = \frac{[F_v(2)-F_v(1)]^{traité}}{[F_v(1)]^{non\ traité}} \quad (Eq.\ 3)$$

**Figure 1.** Comparison of UV-C-induced mortality (as $\log_{10}$ reduction in viable cell number) in three microalgal cultures, *Thalassiosira weissflogii, Heterosigma akashiwo* and *Isochrysis galbanag.*, estimated by culture-based experiments (left) and by staining with FDA and CMFDA (right). Staining was done using protocols described by Steinberg et al. (2011). The FDA staining methodology does not reflect cell viability. Cells were treated with UV-C, held in the dark and then re-dosed with UV-C prior to assay.

**Figure 2.** *Left*: dose-response curves for three microalgal cultures, *Thalassiosira weissflogii, Heterosigma akashiwo* and *Isochrysis galbana*. Cells were treated with UV-C, held in the dark for five days and then re-dosed with UV-C prior to determination of viability by Most Probable Number grow-outs in liquid culture. *Right*: relationships between viability determined from MPN experiments and $F_v$ measured after treatment. $F_v$ has been normalized to the value prior to treatment. Note the interspecific variability and limited dynamic range.

**Fig. 3.** *Left*: dose-response curves for three microalgal cultures, *Thalassiosira weissflogii, Heterosigma akashiwo* and *Isochrysis galbana*. Cells were treated with UV-C, held in the dark for five days and then re-dosed with UV-C prior to determination of viability by Most Probable Number grow-outs in liquid culture. *Right*: relationships between viability determined from MPN experiments and a *Photorepair Index* (PRI), measured immediately after treatment. *PRI* is a dimensionless, fluorescence-based ratio that compares the responses of a microalgal population before and after treatment with UV-C. In this embodiment, it was calculated using Equation 3. Note the similarity of response between species and the relatively wide dynamic range, in contrast to the assays based on vital stains and $F_v$ shown in Fig. 1 and 2.

**Fig. 4.** Example of determination of the input parameters for *PRI* based on sequential incubations at high and low light intensities. $F_v$ is measured on a sample prior to ($T_0$) and after (*Treated*) treatment with UV-C. In each case, a dark-acclimated sample is exposed to high light and a successive period of low light.

**Fig. 5.** Example of determination of the input parameters for *PRI* based on parallel incubations at high light with and without a chloroplastic protein synthesis inhibitor. In this case the antibiotic lincomycin was used as an inhibitor. $F_v$ is measured on a sample prior to (*Untreated*) and after (*Treated*) treatment with UV-C. In each case, a dark-acclimated sample is exposed to high light in parallel incubations with and without the protein synthesis inhibitor.

1) Treatment can be a disinfection protocol with UV-C or control treatment, e.g., without UV-C. A sample is transferred to the Light Reactor after the treatment protocol is complete.

**Treatment**

## Method 1: Light manipulation in series

4) Residence time of organisms in the Dark Treatment reactor (10 - 30 min) is long enough to restore photochemical quenching and relax rapid nonphotochemical quenching.

**Light Reactor**

**Dark Treatment**

3) Throughout the light treatment, water is transferred to the Dark Treatment reactor.

**Detector**

2) Water in the reactor is mixed and conditions are initially dark. Some of the sample is delivered to the Dark Treatment for the $F_v[0]$ measurement, then uniform illumination is applied. The protocol includes a period of time in bright light (e.g., 10 - 120 min. @ 500 - 4000 µmol quanta $m^{-2}$ $s^{-1}$ photosynthetically available radiation, PAR), then a period in dim light (e.g., 30 - 240 min. @ 10 - 50 µmol quanta $m^{-2}$ $s^{-1}$ PAR). Combinations of light intensity and duration are adjusted to maximize system performance.

5) The detector is a configured to measure $F_v$ by one of several possible approaches, including fluorometry, single-cell analysis using imaging, or flow cytometry.

# FIG. 6

## Method 2: Parallel treatment with inhibitor

1) Treatment is the same as in Method 1, but sample is delivered to two Light Reactors.

2a) Light protocol is the same as in Method 1, but an inhibitor of chloroplastic protein synthesis is added to Light Reactor B and darkness is maintained for 20 minutes in both reactors prior to the onset of the light treatment.[1]
2b) Only the high-light treatment is implemented; the rate of photorepair is calculated from the comparison of rates in reactor A vs. B.

3) Dark Treatment is the same as in Method 1.

4a) Detection is the same as in Method 1.
4b) Alternatively, a switching mechanism is employed so flows from A and B can be are analyzed using the same detector.

[1]Alternatively, the dark holding period with or without inhibitor could be in separate containers between the Treatment and Light Reactors.

# FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61963982 B
- WO 2010130031 A, Fraser
- WO 2012061924 A, DaCosta

### Non-patent literature cited in the description

- **SELVIN et al.** *Murphy and Cowles,* 1988
- **ONJI et al.** *Agustí and Sanchez,* 2000
- **GARVEY et al.** *Peperzak and Brussaard,* 2007
- Photoinhibition - a historical perspective. **ADIR, N. ; H. ZER ; S. SHOCHAT ; I. OHAD.** Discoveries in Photosynthesis. Springer, 2005, 931-958
- **AGUSTÍ, S. ; M.C. SANCHEZ.** Cell viability in natural phytoplankton communities quantified by a membrane permeability probe. *Limnol. Oceanogr.,* 2002, vol. 47 (3), 818-828
- **ANDERSON, D. J.** Determination of the lower limit of detection. *Clin. Chem.,* 1989, vol. 35 (10), 2152-2153
- **BLODGETT, R. J.** Serial dilution with a confirmation step. *Food Microbiol.,* 2005, vol. 22 (6), 547-552
- **BLODGETT, R. J.** Upper and lower bounds for a serial dilution test. *J. AOAC Int.,* 2005, vol. 88 (4), 1227-1230
- Appendix 2: Most Probable Number from Serial Dilutions. **BLODGETT, R. J.** Bacteriological Analytical Manual. US Food and Drug Administration, 2010
- **BOLTON, J. R. ; K. G. LINDEN.** Standardization of methods for fluence (UV dose) determination in bench-scale UV experiments. *J. Environ. Eng.-ASCE,* 2003, vol. 129 (3), 209-215
- **BOUCHARD, J.N. ; D.A. CAMPBELL ; S. ROY.** Effects of UV-B radiation on the D1 protein repair cycle of natural phytoplankton communities from three latitudes (Canada, Brazil, and Argentina). *J. Phycol.,* 2005, vol. 41, 273-286
- **BRAND, L. E. ; R. R. L. GUILLARD ; L.S. MURPHY.** A method for the rapid and precise determination of acclimated phytoplankton reproduction rates. *J. Plankton Res.,* 1981, vol. 3, 193-201
- **BOUCHARD, J.N. ; M.L. LONGHI ; S. ROY ; D.A. CAMPBELL ; G. FERREYRA.** Interaction of nitrogen status and UVB sensitivity in a temperate phytoplankton assemblage. *J. Exp. Mar. Biol. Ecol.,* 2008, vol. 359, 67-76
- **CAMPBELL, D. ; V. HURRY ; A.K. CLARKE ; P. GUSTAFSSON ; G. ÖQUIST.** Chlorophyll fluorescence analysis of cyanobacterial photosynthesis and acclimation. *Microbiology and Molecular Biology Reviews,* 1998, vol. 62, 667-683
- **COCHRAN, W.G.** Estimation of bacterial densities by means of the most probable number. *Biometrics,* 1950, vol. 6 (2), 105-116
- **CULLEN, J.J. ; M. ZHU ; D.C. PIERSON.** A technique to assess the harmful effects of sampling and containment for determination of primary production. *Limnol. Oceanogr.,* 1986, vol. 31, 1364-1373
- **DORSEY, J. ; C.M. YENTSCH ; S. MAYO ; C. MCK-ENNA.** Rapid analytical technique for the assessment of cell metabolic activity in marine microalgae. *Cytometry,* 1989, vol. 10, 622-628
- **ETV.** Generic protocol for the verification of ballast water treatment technology. *Program, NSF International for USEPA Environmental Technology Verification Program,* 2010
- Fluorescence induction and relaxation (FIRe) technique and instrumentation for monitoring photosynthetic processes and primary production in aquatic ecosystems. **GORBUNOV, M.Y. ; P.G. FALKOWSKI.** Fundamental Aspects to Global Perspectives. Allen Press, 1029-1031
- **FIRST, M.R. ; L.A. DRAKE.** Approaches for determining the effects of UV radiation on microorganisms in ballast water. *Management of Biological Invasions,* 2013, vol. 4, 87-99
- **FIRST, M.R. ; L.A. DRAKE.** Life after treatment: detecting living microorganisms following exposure to UV light and chlorine dioxide. *J Appl Phycol,* 2013
- **GARVEY, M. ; B. MORICEAU ; U. PASSOW.** Applicability of the FDA assay to determine the viability of marine phytoplankton under different environmental conditions. *Marine Ecology-Progress Series,* 2007, vol. 352, 17-26

- **GENTY, B. ; J.-M. BRIANTAIS ; N.R. BAKER.** The relationship between the quantum yield of photosynthetic electron transport and quenching of chlorophyll fluorescence. *Biochem. Biophys. Acta,* 1989, vol. 990, 87-92
- **GIESKES, W.W.C. ; A.G.J. BUMA.** UV damage to plant life in a photobiologically dynamic environment: The case of marine phytoplankton. *Plant Ecol.,* 1997, vol. 128 (1-2), 16-25
- **GILBERT, F. ; F. GALGANI ; Y. CADIOU.** Rapid assessment of metabolic-activity in marine microalgae - application in ecotoxicological tests and evaluation of water-quality. *Mar. Biol.,* 1992, vol. 112 (2), 199-205
- **KEY, T. ; A. MCCARTHY ; D.A. CAMPBELL ; C. SIX ; S. ROY ; Z.V. FINKEL.** Cell size trade-offs govern light exploitation strategies in marine phytoplankton. *Environmental Microbiology,* 2010, vol. 12, 95-104
- **LIEBICH, V. ; P.P. STEHOUWER ; M. VELDHUIS.** Re-growth of potential invasive phytoplankton following UV-based ballast water treatment. *Aquat. Invasions,* 2012, vol. 7 (1), 29-36
- Using cultures to investigate the physiological ecology of microalgae. **MACINTYRE, H. L. ; J. J. CULLEN.** Algal Culture Techniques. Academic Press, 2005, 287-326
- **MURPHY, A. ; T. COWLES.** Effects of darkness on multi-excitation in vivo fluorescence and survival in a marine diatom. *Limnol. Oceanogr.,* 1997, vol. 42, 1444-1453
- **NEIDHARDT, J. ; J.R. BENEMANN ; L. ZHANG ; A. MELIS.** Photosystem-II repair and chloroplast recovery from irradiance stress: relationship between chronic photoinhibition, light-harvesting chlorophyll antenna size and photosynthetic productivity in Dunaliella salina (green algae). *Photosynth. Res.,* 1998, vol. 56, 175-184
- **ONJI, M. ; T. SAWABE ; Y. EZURA.** An evaluation of viable staining dyes suitable for marine phytoplankton. *Bull Fac Fish Hokkaido Univ,* 2000, vol. 51, 151-158
- **PEPERZAK, L. ; C.P.D. BRUSSAARD.** Flow cytometric applicability of fluorescent vitality probes on phytoplankton. *J. Phycol.,* 2011, vol. 47 (3), 692-702
- **SCHREIBER, U. ; H. HORMANN ; C. NEUBAUER ; C. KLUGHAMMER.** Assessment of photosystem II photochemical quantum yield by chlorophyll fluorescence quenching analysis. *Aust. J. Plant Physiol.,* 1995, vol. 22, 209-220
- **SELVIN, R. ; B. REQUERA ; I. BRAVO ; C.M. YENTSCH.** Use of fluorescein diacetate (FDA) as a single-cell probe of metabolic activity in dinoflagellate cultures. *Bio. Oceanogr.,* 1988, vol. 6, 505-511
- **SINHA, R.P. ; D.-P. HADER.** UV-induced DNA damage and repair: a review. *Photochemical & Photobiological Sciences,* 2002, vol. 1 (4), 225-236
- **STEINBERG, M.K. ; E.J. LEMIEUX ; L.A. DRAKE.** Determining the viability of marine protists using a combination of vital, fluorescent stains. *Mar. Biol.,* 2011, vol. 158, 1431-1437
- The dilution-culture method. **THRONDSEN, J.** Phytoplankton Manual. UNESCO, 1978, 218-224
- **VASILIKIOTIS, C. ; A. MELIS.** Photosystem II reaction center damage and repair cycle: chloroplast acclimation strategy to irradiance stress. *Proc. Nat. Acad. Sci,* 1994, vol. 91, 7222-7226
- **VINCENT, W.F.** Mechanisms of rapid photosynthetic adaptation in natural phytoplankton communities. II. Changes in photochemical capacity as measured by DCMU-induced chlorophyll fluorescence. *J. Phycol.,* 1980, vol. 16, 568-577
- **XIONG, F.** Evidence that UV-B tolerance of the photosynthetic apparatus in microalgae is related to the D1-turnover mediated repair cycle in vivo. *J. Plant Phys.,* 2001, vol. 158, 285-294